# EUROPEAN PATENT APPLICATION

(11) **EP 2 626 110 A1**
(43) Date of publication of application: **14.08.2013**
(21) Application number: 12154385.4
(22) Date of filing: 08.02.2012
(51) Int. Cl.: A61N 1/05

(54) **A thin film for a lead for brain applications**

(71) Applicant: Sapiens Steering Brain Stimulation B.V., 5656 AE Eindhoven (NL)
(72) Inventor: Young, Edward Willem Albert, 6211 LN Maastricht (NL)
(74) Representative: Rupprecht, Kay

(57) **Abstract**

The present invention relates to a thin film (301) for a lead (300) for brain applications with at least one section (150) comprising a high conductive metal (10) and a low conductive metal (20), whereby the low conductive metal (20) is a biocompatible metal (20) and has a lower electrical conductivity than the high conductive metal (10) and whereby the high conductive metal (10) is at least partially encapsuled by the low conductive metal (20). Furthermore, the present invention relates to a method of manufacturing a thin film (301) for a lead (300) for brain applications and a deep brain stimulation system (100).

## Description

The present invention relates to a thin film for a lead for brain applications, to a method of manufacturing a thin film for a lead for brain applications and to a deep brain stimulation (DBS) system.

Implantable neurostimulation devices have been used in the past 10 years to treat acute or chronic neurological conditions. Deep brain stimulation (DBS), the mild electrical stimulation of sub-cortical structures, belongs to this category of implantable devices, and has been shown to be therapeutically effective for Parkinson's disease, dystonia, and tremor. New applications of DBS in the domain of psychiatric disorders (e. g. obsessive compulsive disorder, depression) are being researched and show promising results. In existing systems, the probes are connected to an implantable current pulse generator.

Currently, systems are under development with more, smaller electrodes in a technology based on thin film manufacturing. These are based on thin film(s) that are/is fixed on a carrier material to form a lead. These probes will have multiple electrode areas and will enhance the precision to address the appropriate target in the brain and relax the specification of positioning. Meanwhile, undesired side effects due to undesired stimulation of neighboring areas can be minimized.

Thin films for leads that are based on thin film manufacturing are disclosed e.g. by US 2008/0255439 A1 and have been used in research products in animal studies. These novel systems consist of a lead made from a thin film based on thin technology.

Platinum is a well-accepted metal in medical applications. In particular, deep brain probes are made with platinum wires and distal end metal. However, platinum has a relatively limited electrical conductivity. This poses problems in leads based on thin film manufacturing. As the thin film traces are relatively thin and long in these novel thin film based leads, the electrical resistance of pure platinum traces can be substantial. In particular in thin film designs for wound leads for magnetic resonance (MR) compatibility, as e.g. disclosed in US 2011/224765 A1, the long length of the thin film on the lead would give rise to unacceptably high driving voltages. Additionally, the resistivity of the traces in a thin film should be kept low, typically below 1 to 2 kOhm, to avoid power losses that negatively influence the battery life. However, the traces on the film are narrow.

The materials choice is restricted. Of the accepted metals, platinum as one of few metal or metal alloys has a reasonable conductivity. But in long, narrow, multi trace leads, the resistivity of the traces is a serious concern. The use of thicker platinum layers is not advisable. Thick platinum layers are difficult to handle and process. Therefore, increasing the thickness of the platinum layer to reduce the resistivity of the traces is not a viable option. The use of thicker platinum traces is not feasible. Thick platinum introduces stresses in the thin film and gives rise to pronounced topology on the thin film. Moreover, thick metal limits the bending radius of the thin film probe as materials on top will be stretched.

It is therefore an object of the present invention to provide a thin film for a lead for brain applications having an improved electrical conductivity together with good biocompatible characteristics, in particular to improve the resistivity of the probe, but without compromising on the compatibility of the metal system of the probe.

The above object is solved according to the present invention by a thin film for a lead for brain applications with features of claim 1. Accordingly, a thin film for a lead for brain applications comprises at least one section with a high conductive metal and a low conductive metal, whereby the low conductive metal is a biocompatible metal and has a lower electrical conductivity than the high conductive metal and whereby the high conductive metal is at least partially encapsuled by the low conductive metal.

Consequently, the advantage is achieved that the biocompatibility of the metal system of the probe comprising the high conductive metal and a low conductive metal is not compromised and that the resistivity of the probe is improved. Further advantageously, the electrical conductivity may be increased by maintaining good biocompatible characteristics of the probe. Furthermore, very good magnetic resonance (MR) compatibility can be achieved because the good electrical conductivity enables the use of relatively long spiralled respectively wound thin film. In these leads, the multi trace lead ribbon is spiralled around a carrier.

A low conductive metal (LCM) is a metal with a relatively low electrical conductivity or a metal alloy with a relatively low electrical conductivity, e.g. with an electrical conductivity σ within the range of about 2.00x10⁶ to 9.75x10⁶ S/m, especially between 2.38x10⁶ and 9.43x10⁶ S/m at 20°C. The electrical resistivity p of the low conductive metal (LCM) may be within the range of about 1.00x10⁻⁷ to 4.50x10⁻⁷ Ωm at 20°C, especially between 1.06x10⁻⁷ and 4.20x10⁻⁷ Ωm at 20°C.

A high conductive metal (HCM) is a metal with a relatively high electrical conductivity or a metal alloy with a relatively high electrical conductivity, e.g. with an electrical conductivity σ within the range of about 3.00x10⁷ to 7.00x10⁷ S/m, especially between 3.50x10⁷ and 6.30x10⁷ S/m at 20°C. The electrical resistivity p may be within the range of about 1.50x10-⁸ to 3.00x10⁻⁸ Ωm at 20°C, especially between 1.59x10⁻⁸ and 2.82x10⁻⁸ Ωm at 20°C.

Biocompatible metal in sense of the present invention means e.g. a metal or metal alloy, which has the ability of a biomaterial to perform its desired function with respect to a medical therapy, without eliciting any undesirable local or systemic effects in the recipient or beneficiary of that therapy, but generating the most appropriate beneficial cellular or tissue response in that specific situation, and optimizing the clinically relevant performance of that therapy. With other words, a biocompatible metal in sense of the present invention means e.g. a metal or metal alloy, which is non-toxic to e.g. the brain tissue and/or can be implanted into a human body, preferably into the human brain without or with minor deleterious effects.

The high conductive metal is at least partially outwardly encapsuled by the low conductive metal, so that at least a part of the surface of high conductive metal is covered by the low conductive metal. For example, the surface of high conductive metal can be covered partially by the low conductive metal and furthermore by another material, which is preferably also a biocompatible material.

The lead for brain application is preferably a thin film attached to a carrier.

In a further preferred embodiment the high conductive metal is completely encapsuled by the low conductive metal.

Additionally, it is possible that the high conductive metal and the low conductive metal are at least partially encapsuled by a ceramic material.

Preferably it is possible that the high conductive metal comprises gold and/or copper and/or aluminium and/or silver or is gold or copper or aluminium or silver.

In a further preferred embodiment the low conductive metal comprises platinum and/or titanium and/or titanium nitride or is platinum or titanium or titanium nitride.

In more specific embodiments, a further advantage is that some pair of metals have very good interface adhesion and interdiffusion (i.e. low interdiffusion) properties. For instance, platinum (Pt) and gold (Au) are a good pair. The adhesion of platinum to gold is excellent and inter-diffusion is reported at very high temperatures only and will not compromise the platinum barrier properties and the gold conductivity in the state of the art probe applications probe manufacturing processes. Therefore, platinum is an excellent material to package the gold to prevent gold molecules e.g. from entering the brain tissue. It is therefore preferred that the high conductive metal is gold and that the low conductive metal is platinum.

By this, the further advantage is achieved that the resistivity of e.g. the traces in thin film is improved by introducing a good electrical conductor such as gold as a conductor, but without compromising on the biocompatibility of the metal system by means of preferably an all sided encapsulation of the gold by platinum metal. Thereby and advantageously, low ohmic traces in thin film can be realized by using fully platinum encapsulated gold, which can be of particular importance for wound thin film around a carrier to form a lead. Wounding of a thin film around a carrier is applied to facilitate the bending of the lead and to realize inductance for MR compatibility gives rise to increased length of the cable in order to maintain low resistance in the traces, low Ohmic metallisation on the thin film is required. This embodiment of the present invention enables this advantageously.

In a further preferred embodiment it is possible that the ceramic material comprises SiN, SiOX, Si-Carbid and/or Alumina or that the ceramic is SiN, SiOX, Si-Carbid and/or Alumina.

Preferably, the probe can be at least partially encapsuled by a flexible polymer, whereby the flexible polymer is preferably a biocompatible polymer.

The flexible polymer can be Parylene or epoxy such as SU-8. Alternatively, also silicone, polyimide or polyurethane can be used.

Parylene is a name is for a variety of chemical vapor deposited poly(p-xylylene) polymers used as moisture and dielectric barriers and among these the chosen flexible polymer to be used for the probe can be preferably Parylene-C. Parylene is one of the most biocompatible materials known.

SU-8 is a commonly used epoxy-based negative photoresist. It is a very viscous polymer that can be spun or spread over a thickness ranging from <1 micrometer up to >300 micrometer and still be processed with standard contact lithography. It can be used to pattern high aspect ratio (>20) structures. Its maximum absorption is for ultraviolet light with a wavelength of 365 nm (it is not practical to expose SU-8 with g-line ultraviolet light). When exposed, SU-8's long molecular chains cross-link causing the solidification of the material. SU-8 series photoresists use gamma butyrolactone as the primary solvent.

In a further preferred embodiment it is possible that the flexible polymer is completely encapsuling the low conductive metal and/or preferably also completely encapsuling the ceramic material.

Additionally, an additional layer or pattern can be arranged on the low conductive metal, whereby the layer or pattern preferably comprises titanium or consists of titanium.

Furthermore, the present invention relates to a method of manufacturing a thin film for a lead for brain applications, preferably a thin film for a lead according to any of the claims 1 to 10, the thin film comprising at least one section with a high conductive metal and a low conductive metal, whereby the low conductive metal is a biocompatible metal and has a lower electrical conductivity than the high conductive metal and whereby the high conductive metal is at least partially encapsuled by the low conductive metal.

In a first method option it is possible that the all sided encapsulation of the high conductive metal traces is realized by conducting at least the following process steps:
- sputter depositing of a low conductive metal layer;
- selective high conductive metal plating of traces on the low conductive metal layer through resist mask to form traces;
- patterning of low conductive metal; and
- low conductive metal electroplating of traces.

In a further, alternative method option it is possible that the all sided encapsulation of the high conductive metal traces is realized by conducting at least the following process steps:
- sputter depositing of a low conductive metal layer;
- selective high conductive metal plating on the low conductive metal layer through resist mask, preferably with a negative slope, to form traces;
- sputter deposition of low conductive metal; and
- patterning of low conductive metal.

It is one preferred option it is possible that the high conductive metal is completely encapsuled by the low conductive metal and whereby the high conductive metal is gold, and the low conductive metal is platinum or titanium and whereby the all sided encapsulation of gold traces is realized by conducting at least the following process steps:
- sputter depositing of a platinum layer; a titanium layer may be sputtered prior to the platinum deposition to ensure good adhesion of the platinum;
- selective gold plating of traces on the titanium or platinum layer through resist mask to form traces;
- patterning of platinum, respectively patterning of titanium and platinum; and
- platinum electroplating of traces.

It is another preferred option it is possible that the high conductive metal is completely encapsuled by the low conductive metal and whereby the high conductive metal is gold, and the low conductive metal is platinum or titanium and whereby the all sided encapsulation of gold traces is realized by conducting at least the following process steps:
- sputter depositing of a platinum layer; a titanium layer may be sputtered prior to the platinum deposition to ensure good adhesion of the platinum;
- selective gold plating on the platinum layer through resist mask, preferably with a negative slope, to form traces;
- sputter deposition of platinum; and
- patterning of platinum, respectively patterning of titanium and platinum.

The above mentioned plating of e.g. gold may be an electroplating process. As an alternative to plating, blanket deposition of gold by means of e.g. sputter deposition can be applied. The gold is subsequently masked and etched to form traces. Dry-etching by means of ion milling will enable the formation of trapezium shaped gold traces that can be plated with platinum on the dies walls in a subsequent platinum sputter deposition step.

It is another preferred option it is possible that the high conductive metal is completely encapsuled by the low conductive metal and whereby the high conductive metal is gold, and the low conductive metal is platinum and whereby the all sided encapsulation of gold traces is realized by conducting at least the following process steps:
- sputter depositing of a platinum layer;
- sputter depositing of gold on the platinum layer and subsequently structure the gold to form traces, preferably with sloped edges;
- sputter deposition of platinum; and
- patterning of titanium or platinum.

Still further, the present invention relates to a deep brain stimulation system comprising at least on probe according to one of claims 1 to 11 or comprising at least on probe being manufactured acccording to method of any one of claims 12 to 14.

Further details and advantages of the present invention shall be described hereinafter with respect to the drawings:
Fig. 1: a schematical drawing of a neurostimulation system for deep brain stimulation (DBS);
Fig. 2: a further schematical drawing of a probe neurostimulation system for deep brain stimulation (DBS) and its components;
Fig. 3: a schematical drawing of a probe system according to the present invention;
Fig. 4a, b: a schematical drawing of steps of an alternative of the manufacturing process;
Fig. 5a, b: a further schematical drawing of steps of a further alternative of the manufacturing process; and
Fig. 6: a schematical drawing of sections of a thin film of a lead.

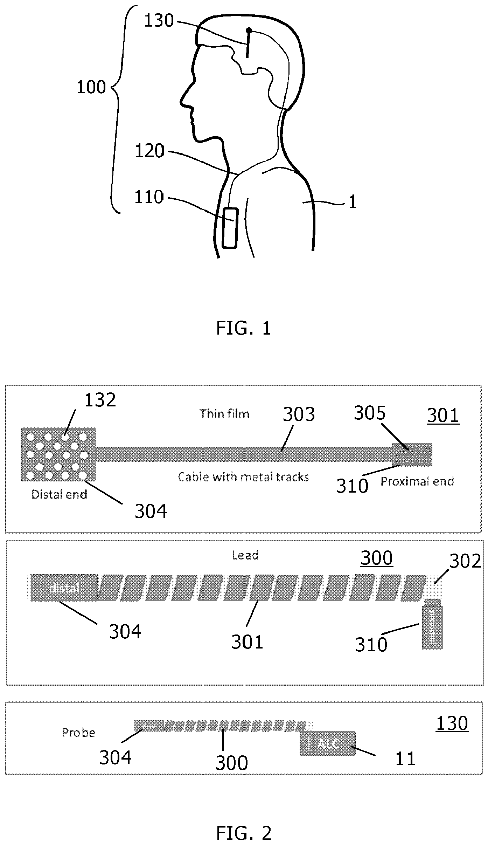

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Connector (ALC) element 11 comprising electronic means to address electrodes 132 on the distal end 304 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 on the lead 300 is electrically connected to the ALC element 11. The ALC element 11 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises of metal lines (not shown) to connect each distal electrodes 132 to a designated proximal contact 305.

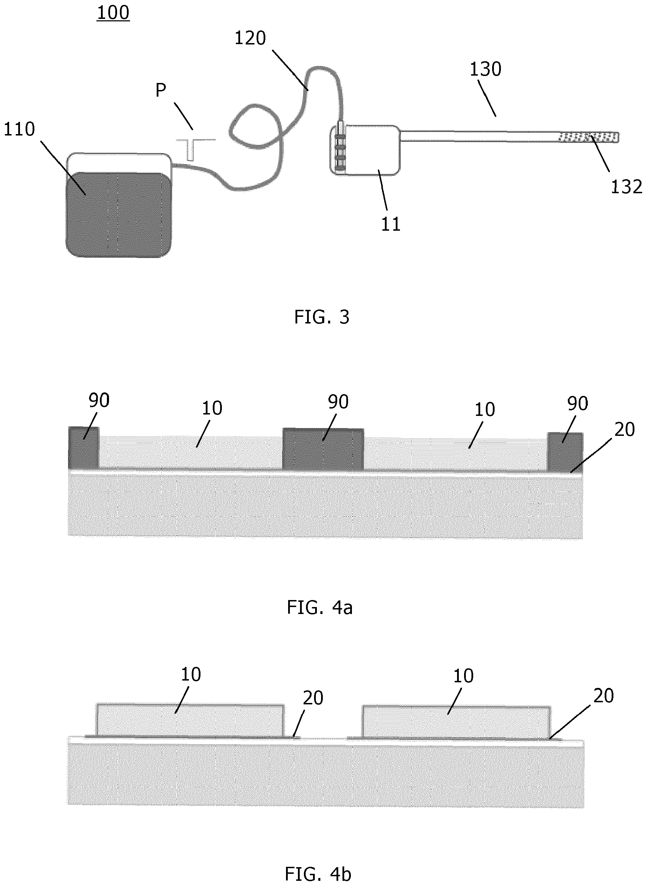

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the ALC 11. The controller 110 can be an implantable pulse generator (IPG) 110.

According to the present invention, the resistivity of the traces in a thin film 301 are now kept low, typically below 1 to 2 kOhm, to avoid the use of high driving voltages and avoid power losses that negatively influence the battery life. According to the present invention the resistivity of the traces in thin film lead by introducing a high conductive metal (HCM) such as copper (Cu), aluminium (AI), gold (Au) or silver (Ag) as a conductor may be improved, but without compromising on the compatibility of the metal system by applying an all sided encapsulation of the HCM with a biocompatible metal such as platinum (Pt) or titanium (Ti). Alternatively, also a metal compound such as titanium nitride (TiN) can be used.

Gold, silver and aluminium are not sufficiently biocompatible and are not generally accepted for chronic use in brain probes. All sided encapsulation of the HCM with a biocompatible metal or metal compound/alloy, namely the biocompatible low conductive metal (LCM) may ensure that the tissue is never exposed to the non-biocompatible respectively less-biocompatible HCM.

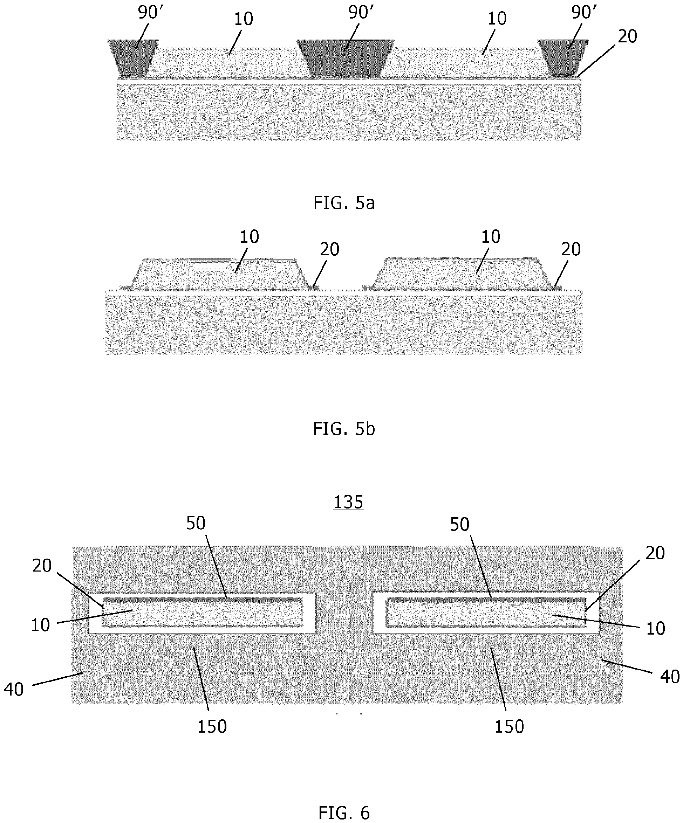

The encapsulation with biocompatible LCM can be combined with additional encapsulation by ceramics such as SiN, SiOx, Si-Carbide and Alumina as e.g. shown in Figure 6 and described in detail hereinafter.

These HCM such as gold show much better electrical conductivity. Some of the HCM show e.g. roughly six times better electrical conductivity than platinum. Therefore, HCM layers can be up to six times thinner for the same resistivity.

Standard vacuum thin film deposition technologies, such as sputter deposition, can be applied to deposit the HCM. Alternatively, deposition of the relatively thick layers of these HCMs can be performed by electroplating. The use of electroplated e.g. gold for application in low ohmic traces in implants is already known.

One preferred embodiment of this invention is the use of a HCM with an all sided coating with a low-conductive but biocompatible metal (LCM) while maintaining the benefits of said LCM as a safe metal in brain probe applications. The all sided encapsulation of the HCM by a LCM prevents exposure of the HCM to tissue under all circumstances. The LCM prevents the diffusion of HCM into the thin film encapsulation materials and subsequent diffusion into the brain tissue.

The encapsulation with biocompatible metal can be combined with additional encapsulation by ceramics such as SiN, Siox, Si-Carbide and Alumina.

In a preferred embodiment of the invention, the HCM is gold (Au), and the LCM is platinum (Pt). All sided encapsulation of gold traces can be realized by following the following manufacturing process (A) shown in Figure 4a and 4b and by conducting the following process steps:
- sputter depositing of a platinum 20 layer;
- selective gold 10 electroplating of traces on the platinum 20 layer through resist mask 90 to form traces;
- patterning of platinum 20; and
- platinum 20 electroplating of traces.

An additional titanium layer can be applied to enhance the adhesion of platinum.

Alternatively, the following manufacturing process (B) can be applied to create all side encapsulated gold traces shown in Figure 5a and 5b and by conducting at least the following process steps:
- sputter depositing of a platinum layer 20;
- selective gold 10 plating on the platinum layer through resist mask 90' with a negative slope to form traces;
- sputter deposition of platinum; and
- patterning of platinum.

In order to enhance the protection of the metal traces, the traces can have an additional encapsulation by a ceramic material 30 such as SiN, SiOxide, SiC or Alumina. This material can be applied in stacks or mixtures. Well known deposition processes that may be used are e.g. Low Pressure Chemical Vapour Deposition (LPCVD), Plasma Enhanced Chemical Vapour Deposition (PECVD) and Atomic Layer Deposition (ALD).

It is possible that only the trace part of the thin film 301 of the lead 300 is equipped with sections with a HCM and a LCM and that the distal electrodes 132 can be manufactured without gold in the thin film layer stack. Thereby and advantageously, any risk of exposure of gold to brain tissue in the distal area of the lead 300 is prevented.

As can be seen in Figure 6, only the trace part 135 of the thin film 301 of the lead 300 is equipped with sections 150 with a HCM 10, i.e. gold 10 and a LCM 20, i.e. platinum 20. The HCM 10 gold is completely encapsuled by the LCM 20 platinum. An additional pattern 50 is arranged on the LCM 20, whereby the pattern 50 consists of titanium. Additionally, the HCM 10 and the LCM 20 are completely encapsuled by a ceramic material 30, which is in the embodiment shown in Figure 6 a mixture of SiOx and SiNx. Both sections 150 are fully encapsuled by a flexible and biocompatible polymer 40, which is in this embodiment Parylene.

In the distal part, sections of the parylene and the ceramic coating are removed to expose the biocompatible metal. These are the electrode areas. The electrode can consist of the full metal stack, the LCM and the HCM. However, the electrodes are relatively small and the electrical conductivity of the LCM alone is sufficient in this area. Therefore, the distal end of the probe can consist of LCM only.

Explicitly disclosed together with the above described invention is also a method of using a probe 130 comprising a lead 300 according to the present invention for brain applications and treatments, e.g. for DBS. Preferably, at least one thin film 301 according to any of the claims 1 to 10 or at least one thin film 301 for a lead 300 manufactured according to the method of claims 11 to 14 is used.

Furthermore, explicitly disclosed together with the above described invention is also a method of using a deep brain stimulation system 100 according to the present invention for brain applications and treatments, e.g. for deep brain stimulation. Preferably, the deep brain stimulation system 100 of claim 15 is used (see also Figure 3).

## Claims

1. A thin film (301) for a lead (300) for brain applications with at least one section (150) comprising a high conductive metal (10) and a low conductive metal (20), whereby the low conductive metal (20) is a biocompatible metal (20) and has a lower electrical conductivity than the high conductive metal (10) and whereby the high conductive metal (10) is at least partially encapsuled by the low conductive metal (20).

2. The thin film (301) according to claim 1, **characterized in that** the high conductive metal (10) is completely encapsuled by the low conductive metal (20) and/or that the high conductive metal (10) and the low conductive metal (20) are at least partially encapsuled by a ceramic material (30).

3. The thin film (301) according to any of the preceding claims, **characterized in that** the high conductive metal (10) comprises gold and/or copper and/or aluminium and/or silver or is gold or copper or aluminium or silver.

4. The thin film (301) according to any of the preceding claims, **characterized in that** the low conductive metal (20) comprises platinum and/or titanium and/or titanium nitride or is platinum or titanium or titanium nitride.

5. The thin film (301) according to claim 3 and 4, **characterized in that** the high conductive metal (10) is gold and that the low conductive metal (20) is platinum.

6. The thin film (301) according to any of claims 2 to 5, **characterized in that** the ceramic material (30) comprises SiN, SiOX, Si-Carbid and/or Alumina or that the ceramic is SiN, SiOX, Si-Carbid and/or Alumina.

7. The thin film (301) according to any of the preceding claims, **characterized in that** the probe (131) is at least partially encapsuled by a flexible polymer (40), whereby the flexible polymer (40) is preferably a biocompatible polymer.

8. The thin film (301) according to claim 7, **characterized in that** the flexible polymer (40) is Parylene or SU-8 or silicone or polyimide or polyurethane.

9. The thin film (301) according to claims 7 or 8, preferably according to claims 7 or 8 and one of claims 2 to 6, **characterized in that** the flexible polymer (40) is completely encapsuling the low conductive metal (20) and/or preferably also completely encapsuling the ceramic material (30).

10. The thin film (301) according to any of the preceding claims, **characterized in that** an additional layer or pattern (50) is arranged on the low conductive metal (20), whereby the layer or pattern (50) preferably comprises titanium or consists of titanium.

11. A method of manufacturing a thin film (301) for a lead (300) for brain applications, preferably a thin film (301) for a lead (300) according to any of the claims 1 to 10, the thin film (301) comprising at least one section (150) with a high conductive metal (10) and a low conductive metal (20), whereby the low conductive metal (20) is a biocompatible metal (20) and has a lower electrical conductivity than the high conductive metal (10) and whereby the high conductive metal (10) is at least partially encapsuled by the low conductive metal (20).

12. The method according to claim 11, **characterized in that** the high conductive metal (10) is completely encapsuled by the low conductive metal (20) and whereby the high conductive metal (10) is gold, and the low conductive metal (10) is platinum and whereby the all sided encapsulation of gold traces is realized by conducting at least the following process steps:
- sputter depositing of a platinum layer;
- selective gold plating of traces on the platinum layer through resist mask (90) to form traces;
- patterning of platinum; and
- platinum electroplating of traces.

13. The method according to claim 12, **characterized in that** the high conductive metal (10) is completely encapsuled by the low conductive metal (20) and whereby the high conductive metal (10) is gold, and the low conductive metal (10) is platinum and whereby the all sided encapsulation of gold traces is realized by conducting at least the following process steps:
- sputter depositing of a platinum layer;
- selective gold plating on the platinum layer through resist mask (90'), preferably with a negative slope, to form traces;
- sputter deposition of platinum; and
- patterning of platinum.

14. The method according to claim 12, **characterized in that** the high conductive metal (10) is completely encapsuled by the low conductive metal (20) and whereby the high conductive metal (10) is gold, and the low conductive metal (10) is platinum and whereby the all sided encapsulation of gold traces is realized by conducting at least the following process steps:
- sputter depositing of a platinum layer;
- sputter depositing of gold on the platinum layer and subsequently structure the gold to form traces, preferably with sloped edges;
- sputter deposition of platinum; and
- patterning of titanium or platinum.

15. A deep brain stimulation system (100) comprising at least one lead (300) with a thin film (301) according to one of claims 1 to 10 or comprising at least one thin film (301) being manufactured acccording to method of any one of claims 11 to 14.
